# EUROPEAN PATENT SPECIFICATION

(11) **EP 3 082 584 B1**
(45) Date of publication and mention of the grant of the patent: **21.10.2020**
(21) Application number: 14870684.9
(22) Date of filing: 25.08.2014
(51) Int. Cl.: A61B 5/02, A61B 5/0205, A61B 5/024, A61B 5/11

(54) **APPARATUS FOR MEASURING BIO-INFORMATION AND A METHOD FOR ERROR COMPENSATION THEREOF**
VORRICHTUNG ZUR MESSUNG VON BIOINFORMATIONEN UND VERFAHREN ZUR FEHLERKOMPENSATION DAFÜR
APPAREIL DE MESURE DE BIO-INFORMATIONS ET PROCÉDÉ DE COMPENSATION D'ERREUR DE CELUI-CI

(30) Priority: 18.12.2013 KR 20130158169; 24.12.2013 KR 20130162325
(43) Date of publication of application: 26.10.2016
(73) Proprietor: LG Electronics Inc., Yeongdeungpo-gu Seoul 07336 (KR)
(72) Inventor: YOON, Hyoung Kil, Seoul 150-721 (KR); JO, In Ah, Seoul 150-721 (KR); JIN, Won Hyeog, Seoul 150-721 (KR)
(74) Representative: Ter Meer Steinmeister & Partner
(86) International application number: PCT/KR2014/007864
(87) International publication number: WO 2015/093716

(56) References cited:
- EP-A1- 1 297 784
- WO-A1-2010/007589
- JP-A- 2002 320 591
- JP-A- 2002 500 768
- JP-A- 2004 503 282
- JP-A- 2009 519 737
- JP-A- 2011 156 376
- JP-A- 2011 217 822
- KR-A- 20130 084 993
- US-A1- 2004 236 227
- US-A1- 2008 097 221
- US-A1- 2012 306 643
- US-A1- 2012 316 455

## Description

### Technical Field

The present disclosure relates to an apparatus for measuring bio-information and a method for error compensation thereof.

### Background Art

A conventional wearable activity tracker may be classified into two types. That is, a band type product made of elastic materials such as band type silicone or rubber, and a wristwatch type product. The former is wrist-unattachable and equipped only with a pedometer function mounted with an acceleration sensor, while the latter is equipped with an optical heart rate sensor to provide health information of a wearer such as heart rate activity state in addition to pedometer function.

Meanwhile, the optical pulse sensor estimates heart rate activities by measuring blood flowing in the blood vessel using an optical characteristic of bio tissues. To be more specific, PPG (photoplethysmogram) observes optical characteristics of bio tissues such as light reflectivity, absorptance and transmittance that show during volumetric change in blood vessel by using a light, and measures the heart rate using the change. The method of non-invasive method is widely used due to enablement of measuring bio signals, advantageous because of miniaturized size and convenience in usage, and conducive to development of wearable life signal detection sensor.

The optical heart rate sensor includes a light source and a light receiver, where when a light is incident on skin from the light source, and a light reflected from the incident light is collected by the light receiver, the number of heart rates can be detected from changes in quantity of the collected light.

US 2012/0306643 A1 describes a system that includes a wrist band having one or more sensors that measure the heart rate or other biometric data of a user wearing the band; and a display on the band that displays a message based on biometric data measured by the band. In one example, the band includes an accelerometer.

WO 2010/007589 A1 describes a medical device having at least one sensor, such as a heart pulse sensor or a sensor for measuring acceleration. The medical device can be worn as a watch.

EP 1297784 A1 describes a device for detecting the pulse rate. The measuring principle includes measuring the intensity of radiant energy after propagation of light through the human body tissue by means of four light detectors. A motion detecting device provides a motion reference signal. The input signals are processed in order to remove motion-related contributions due to motion of the detecting device on and with respect to the human body tissue.

US 2004/0236227 A1 describes a portable instrument for measuring a physiological quantity, having an optical element forming a guide coupled to a light source for distributing light emission into several determined illumination zones. In an example, the same principle is applied to a detection device.

KR 2013 0084993 A describes a wearable device assembly with an exercise function. The device assembly has a control unit having one or more sensors. The control unit detects the activity of a user. The control unit selectively radiates a display unit system.

### Disclosure of Invention

### Technical Problem

However, the conventional optical heart rate sensor is disadvantageous in that only one PH (Photo diode) is available, a light source takes a shape of a point light source, and heart rate can be measured by being attached to skin. Thus, the conventional optical heart rate sensor is applicable only to a wrist watch type product, and is relatively difficult to be applied to a wrist-unattachable, band type product.

### Solution to Problem

The invention is indicated in the independent claims. Further embodiments are indicated in the dependent claims.

Exemplary aspects of the present disclosure are to substantially solve at least the above problems and/or disadvantages and to provide at least the advantages as mentioned below. Thus, the present disclosure is directed to provide an apparatus for measuring bio-information and a method for error compensation therefor.

In one general aspect of the present invention, there is provided an apparatus for measuring bio-information, the apparatus comprising:
a heart rate sensor unit configured to measure heart rates by receiving a light that has entered and come out from skin;
an acceleration sensor unit configured to output a step count by measuring the step count of a wearer;
a display unit configured to display the measured heart rates and step count; and
a wrist-wearable connection unit configured to electrically connect the heart rate sensor, the acceleration sensor unit and the display unit.

The heart rate sensor unit includes,
a light source unit configured to emit a linear light source,
a light receiving unit configured to receive the light that has entered and come out from the skin from the light emitted from the light source, and
a controller configured to detect the heart rates from a quantity of light received by the light receiving unit.

Preferably, but not necessarily, the light source unit may include,
an LED (Light Emitting Diode) configured to emit a light of point light source,
a curved light guide of a particular curvature radius configured to advance the emitted light to a particular direction,
a plurality of V-shaped patterns configured to emit a light to a particular direction by refracting the emitted light, and
a reflective plate configured to reflect a light emitted to an outside from the light guide into an interior of the light guide.

Preferably, but not necessarily, the LED may use a yellowish green color light source.

The light receiving unit includes,
a first light receiving unit arranged at an upper left surface based on a lengthwise direction of the light source,
a second light receiving unit arranged at an upper right surface based on a lengthwise direction of the light source,
a third light receiving unit arranged at a bottom left surface based on a lengthwise direction of the light source, and
a fourth light receiving unit arranged at a bottom right surface based on a lengthwise direction of the light source.

Preferably, but not necessarily, the light receiving unit may include,
a photodetector configured to receive a light that has entered and come out of skin from a light emitted from the light source unit, and
a light receiving house configured to wrap the photodetector and to gradually broaden at an entrance toward a skin contact surface.

Preferably, but not necessarily, the plurality of V-shaped patterns may taper off at a spacing of adjacent patterns as being distanced from the LED.

Preferably, but not necessarily, the connection unit may be a wrist band type connector unit of elastic material.

Preferably, but not necessarily, the connection unit may be a wrist watch type, wrist-attachable connector unit.

In another general aspect of the present disclosure, there is provided a method for error compensation in a heart rate sensor including a first light receiving unit arranged at an upper left surface of a line light source, a second light receiving unit arranged at an upper right surface of the line light source,
a third light receiving unit arranged at a bottom left surface of the line light source, and a fourth light receiving unit arranged at a bottom right surface of the line light source, the method comprising:
detecting an error by comparing light signals received by the first to fourth light receiving units;
multiplying a predetermined weight to a greater light signal as a result of comparison of the light signals when the error is generated; and
adding a size of a light signal multiplied by the weight to a size of a light signal not multiplied by the weight.

Preferably, but not necessarily, the error may include a first error, which is a difference between a first signal, which is a sum of light signals received by the first and second light receiving units and a second signal, which is a sum of light signals received by the third and fourth light receiving units,
a second error, which is a difference between a third signal, which is a sum of light signals received by the first and third light receiving units and a fourth signal, which is a sum of light signals received by the second and fourth light receiving units, and
a third error, which is a difference between a fifth signal, which is a sum of light signals received by the first and fourth light receiving units and a sixth signal, which is a sum of light signals received by the second and third light receiving units.

Preferably, but not necessarily, the step of multiplying a predetermined weight to a greater light signal as a result of comparison of the light signals when the error is generated may include,
multiplying a predetermined weight to a greater signal between the first and second signals when the first error is generated,
multiplying a predetermined weight to a greater signal between the third and fourth signals when the second error is generated, and
multiplying a predetermined weight to a greater signal between the fifth and sixth signals when the first error is generated.

Preferably, but not necessarily, the method may further comprise determining a greater value as a heart rate reference signal between a size of an optical signal multiplied by the weight and a size of an optical signal not multiplied by the weight.

In still another general aspect of the present disclosure, there is provided a method for error compensation in a heart rate sensor including a first light receiving unit arranged at an upper left surface of a line light source, a second light receiving unit arranged at an upper right surface of the line light source,
a third light receiving unit arranged at a bottom left surface of the line light source, and a fourth light receiving unit arranged at a bottom right surface of the line light source, the method comprising:
detecting an error by comparing light signals received by the first to fourth light receiving units;
multiplying a predetermined weight to a light signal having the greatest value as a result of comparison of the light signals when the error is generated; and
adding a size of a light signal multiplied by the weight to a size of a light signal not multiplied by the weight.

Preferably, but not necessarily, the step of detecting an error by comparing light signals received by the first to fourth light receiving units may include determining a light receiving unit having received a largest size of optical signal by comparing a first error, which is a difference between a first signal, which is a sum of light signals received by the first and second light receiving units and a second signal, which is a sum of light signals received by the third and fourth light receiving units, a second error, which is a difference between a third signal, which is a sum of light signals received by the first and third light receiving units and a fourth signal, which is a sum of light signals received by the second and fourth light receiving units, and a third error, which is a difference between a fifth signal, which is a sum of light signals received by the first and fourth light receiving units and a sixth signal, which is a sum of light signals received by the second and third light receiving units.

### Advantageous Effects of Invention

The present disclosure has an advantageous effect in that heart rate can be accurately measured using a line light source instead of point light source, even if the apparatus for measuring the bio-information is not fully attached to a wrist.

Another advantageous effect is that the light receiving efficiency can be greatly enhanced using four light receiving units.

Still another advantageous effect is that the present invention can be applied to both the wrist band type connector unit and a wrist watch typed connector unit.

Still further advantageous effect is that a heart rate signal can be stably detected even if a heart rate sensor including a plurality of light receiving units is not attached to skin.

Still further advantageous effect is that the present invention can choose an appropriate method between a comparative signal weighting method and an absolute signal weighting method in consideration of weight setting, SNR (Signal-to-Noise-Ratio) and operation environment of heart rate sensor.

### Brief Description of Drawings

FIG.1 is a block diagram illustrating a configuration of an apparatus for measuring bio-information according to an exemplary embodiment of the present disclosure.
FIG. 2 is a block diagram illustrating a configuration of a heart rate sensor unit according to an exemplary embodiment of the present disclosure.
FIG. 3 is a schematic view illustrating arrangement of a light source unit of a heart rate sensor unit, and first to fourth light receiving units according to an exemplary embodiment of the present disclosure.
FIG. 4 is a schematic view illustrating a structure of a light source unit according to an exemplary embodiment of the present disclosure.
FIGS. 5 and 6 are respectively a perspective view and a bottom view of a structure of a light source unit according to an exemplary embodiment of the present disclosure.
FIG. 7 is a plan view illustrating structures of light source unit of heart rate sensor unit, and first to fourth light receiving units according to an exemplary embodiment of the present disclosure.
FIG. 8 is a lateral view illustrating structures of first to fourth light receiving units according to an exemplary embodiment of the present disclosure.
FIG. 9 is a schematic view illustrating a heart rate sensor unit attached to skin of an apparatus for measuring bio-information according to an exemplary embodiment of the present disclosure.
FIG. 10 is a schematic view illustrating a structure of an apparatus for measuring bio-information according to an exemplary embodiment of the present disclosure.
FIG. 11 is a schematic view illustrating types of alignment errors to be considered when a method for error compensation according to an exemplary embodiment of the present disclosure is performed.
FIG. 12 is a schematic view illustrating a method for error compensation according to an exemplary embodiment of the present disclosure.

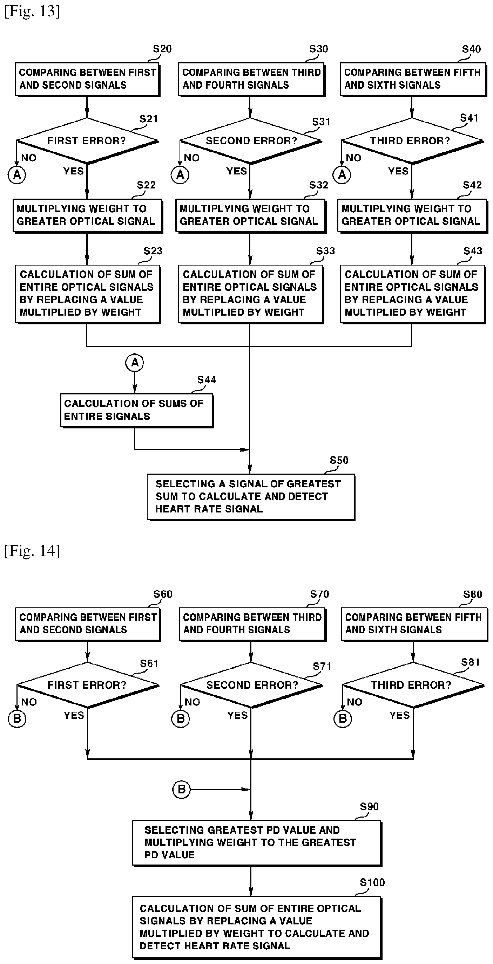

FIG. 13 is a schematic view illustrating a comparative signal weighting method in a method for error compensation according to an exemplary embodiment of the present disclosure.
FIG. 14 is a schematic view illustrating an absolute signal weighting method in a method for error compensation according to an exemplary embodiment of the present disclosure.

### Mode for the Invention

Various exemplary embodiments will be described more fully hereinafter with reference to the accompanying drawings, in which some exemplary embodiments are shown. The present inventive concept may, however, be embodied in many different forms and should not be construed as limited to the example embodiments set forth herein. Rather, the described aspect is intended to embrace all such alterations, modifications, and variations that fall within the scope and novel idea of the present disclosure.

Now, an exemplary embodiment of the present disclosure will be described in detail with reference to the accompanying drawings.

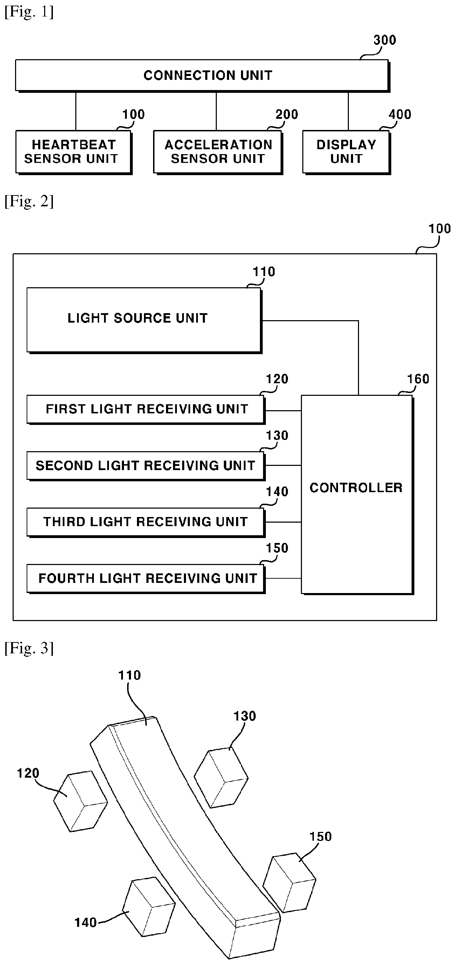

FIG.1 is a block diagram illustrating a configuration of an apparatus for measuring bio-information according to an exemplary embodiment of the present disclosure.

Referring to FIG. 1, an apparatus for measuring bio-information (hereinafter referred to as) may include a heart rate sensor unit (100), an acceleration sensor unit (200), a wrist-wearable connection unit (300) and a display unit (400).

The heart rate sensor unit (100) may include an optical heart rate sensor to display a heart rate (pulse) of a wearer on the display unit (400) by measuring the heart rate of the wearer. The heart rate sensor unit (100) uses a principle, in which a light that has entered and come out from the skin from the light emitted from a light source is changed by heart rate in time in terms of optical absorption degree in response to plastid in blood such as hemoglobin available in skin tissue and blood, where the heart rate sensor unit (100) receives a light returned by the light receiving unit of the heart rate sensor unit (100) and detects the heart rate by converting the received light to an electrical signal.

The acceleration sensor unit (200) may measure an acceleration of an apparatus for measuring bio-information (apparatus) according to an exemplary embodiment of the present disclosure and provide a pedometer function. The acceleration sensor unit (200) may include an acceleration sensor mounted on a pedometer or a walk-step measuring device, measure an acceleration speed in response to movement of a wearer of the apparatus, and display on the display unit (400) a step count by converting the measured acceleration speed to the step count.

The connection unit (300) functions to connect the heart rate sensor unit (100), the acceleration sensor unit (200) and the display unit (400), and may take a shape of being wearable on a wrist, a fore arm, an upper arm, a thigh, a head and/or a finger. The connection unit (300) may be formed in the shape of a band type connection unit of elastic material such as silicone and a rubber, may be formed in the shape of a wrist watch type connection unit wearable to be attached to a wrist like an wrist watch, or may be formed in the shape of a head band type connection unit wearable to be attached to a head like a head band.

The display unit (400) may display heart rate information measured by the heart rate sensor unit (100). Furthermore, the display unit (400) may display pedometer information measured by the acceleration sensor unit (200).

FIG. 2 is a block diagram illustrating a configuration of a heart rate sensor unit (100) according to an exemplary embodiment of the present disclosure.

Referring to FIG. 2, the heart rate sensor unit (100) according to an exemplary embodiment of the present disclosure may include a light source unit (110), first to fourth light receiving units (120, 130, 140, 150) and a controller (160).

The light source unit (110) emits a light. The light source unit (110) may use an optical light guide for emitting a line light source. The use of line light source may irradiate a light on a broader skin area than that of point light source, whereby a sufficient quantity of light can be incident on a wrist area even if the apparatus is not closely attached to the wrist. A detailed structure of the light source unit (110) will be described with reference to FIGS. 3 to 6 later.

The first to fourth light receiving units (120, 130, 140, 150) may be symmetrically (vertically and horizontally) arranged based on lengthwise direction of the light source unit (110), and may receive a light emitted from the light source unit (110) that has entered and come out from the skin. The arrangement of the first to fourth light receiving units (120, 130, 140, 150) may be changed depending on a length and an area of the light source unit (110).

The first to fourth light receiving units (120, 130, 140, 150) may include first to fourth photodetectors (121, 131, 141, 151), and the first to fourth photodetectors (121, 131, 141, 151) may be PDs (Photo Diodes). The first to fourth light receiving units (120, 130, 140, 150) may transmit the received light to the controller (160) by converting the received light to an electrical signal.

The controller (160) may detect quantity of light emitted from the light source unit (110) and may receive the light received by the first to fourth light receiving units (120, 130, 140, 150) in electrical signal.

FIG. 3 is a schematic view illustrating arrangement of the light source unit (110) of the heart rate sensor unit (100), and the first to fourth light receiving units(120, 130, 140, 150) according to an exemplary embodiment of the present disclosure.

Referring to FIG. 3, the light source unit (110) may be a line light source unit configured to take a shape of a curved surface. When a curved line light source is employed, the light source unit can be attached to a measurement unit having a curve as in the wrist. The first light receiving unit (120) may be arranged at an upper left surface based on a lengthwise direction of the light source, the second light receiving unit (130) may be arranged at an upper right surface based on a lengthwise direction of the light source, the third light receiving unit (140) may be arranged at a bottom left surface based on a lengthwise direction of the light source, and the fourth light receiving unit (150) may be arranged at a bottom right surface based on a lengthwise direction of the light source.

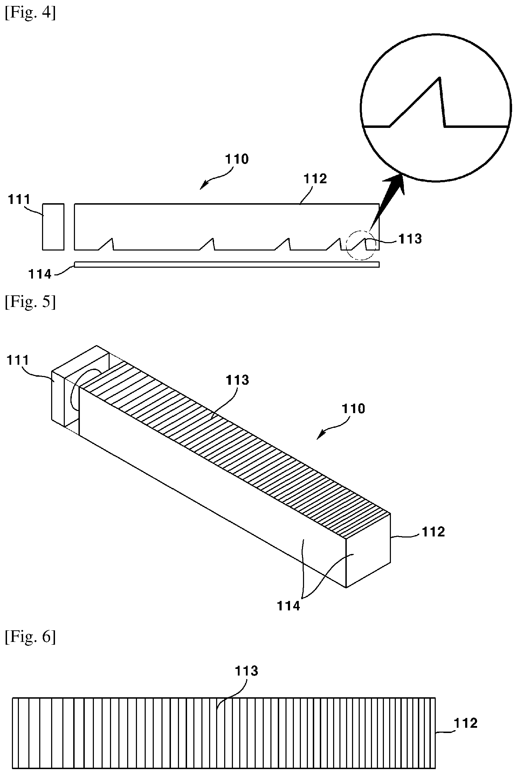

FIG. 4 is a schematic view illustrating a structure of the light source unit (110) according to an exemplary embodiment of the present disclosure.

Referring to FIG. 4, although the light source unit (110) may be preferably a line light source unit configured to take a shape of a curved surface, the light source unit is illustrated herein as a line light source unit having no curvature, for convenience sake.

The light source unit may include an LED (Light Emitting Diode, 111), a curved light guide (112), a plurality of V-shaped patterns (113) and a reflective plate (114).

The LED (111) may emit a light of point light source shape to a lengthwise direction of the light source unit (110). Although only one LED (111) is illustrated at a distal end in FIG. 4D, a total of two LEDs may be arranged each at a distal end.

Although the LED (111) uses a wavelength of red or an infrared region, the LED (111) preferably uses a yellowish green color light source in consideration of optical characteristic of skin tissue. The curved light guide (112) is preferred to have a particular curvature radius of about 20mm to allow being attached to a radial artery of the wrist when the heart rate sensor unit (100) is brought into contact with the wrist. The curved light guide (112) may be formed with a flexible material.

The plurality of V-shaped patterns (113) may taper off at a spacing of adjacent patterns as being distanced from the LED (111), which is to emit as much as uniform refracted light because the quantity of reached light decreases as being distanced from the light source.

The reflective plate (114) may be arranged on all surfaces except for a direction from which the light is emitted from the curved light guide (112) to reflect the light into an interior of the light guide, whereby the light loss can be minimized.

FIGS. 5 and 6 are respectively a perspective view and a bottom view of a structure of a light source unit according to an exemplary embodiment of the present disclosure.

Referring to FIGS. 5 and 6, although the light source unit (110) may be preferably a line light source unit configured to take a shape of a curved surface, the light source unit is illustrated herein as a line light source unit having no curvature, for convenience sake.

The plurality of V-shaped patterns (113) may taper off at a spacing of adjacent patterns as being distanced from the LED (111), which is to emit as much uniform refracted light as possible, because the quantity of reached light decreases as being distanced from the light source.

The curved light guide (112) is formed with a plurality of V-shaped patterns (113) to minimize the quantity of leaked light by arranging the reflective plate (114) on all surfaces except for a direction from which the light is emitted.

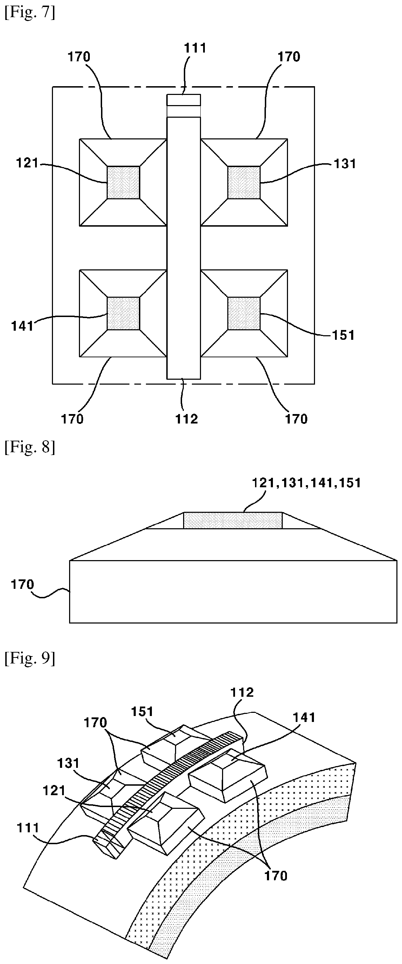

FIG. 7 is a plan view illustrating structures of light source unit (110) of heart rate sensor unit (100), and first to fourth light receiving units (120, 130, 140, 150) according to an exemplary embodiment of the present disclosure.

Referring to FIG. 7, the first to fourth light receiving units (120, 130, 140, 150) may be symmetrically (vertically and horizontally) arranged based on lengthwise direction of the light source unit (110).

Each of the first to fourth light receiving units (120, 130, 140, 150) may take a combined shape of first to fourth photodetectors (121, 131, 141, 151) and a light receiving housing (170). The first to fourth photodetectors (121, 131, 141, 151) may be PDs (Photo Diodes) configured to receive light emitted from the light source unit that has entered and come out of the skin.

The light receiving housing (170) may be surface-treated with a hollow material of high reflectivity to take a shape of wrapping the first to fourth photodetectors (121, 131, 141, 151). When the PD is directly arranged on the surface of skin using no light receiving housing, the light receiving rate of light that has entered and come out of the skin may decrease, such that the light receiving housing (170) may be employed to collect as much light as possible.

The PD may be positioned at an area inside the light receiving housing distanced at a predetermined space from the skin. The light receiving housing (170) may take a shape of a bottomless quadrangular pyramid.

FIG. 8 is a lateral view illustrating structures of first to fourth light receiving units (120, 130, 140, 150) according to an exemplary embodiment of the present disclosure.

Referring to FIG.8, the first to fourth light receiving units (120, 130, 140, 150) may include the first to fourth photodetectors (121, 131, 141, 151) and the light receiving housing (170).

The light receiving housing (170) may have a hollow inside to prevent from being directly contact to the skin and take a shape of wrapping the first to fourth photodetectors (121, 131, 141, 151) to collect the light that has entered and come out from the skin.

Referring to FIG.8, the light receiving housing (170) may take a shape of a hollow and bottomless quadrangular pyramid to include the first to fourth photodetectors (121, 131, 141, 151), the present disclosure is not limited thereto, and the light receiving housing (170) may take a shape of any configuration capable of collecting light and being attached to the skin, such as a circular truncated cone, a cube and/or a rectangular parallelepiped, each being less a bottom surface.

FIG. 9 is a schematic view illustrating a heart rate sensor unit (100) attached to skin of an apparatus for measuring bio-information according to an exemplary embodiment of the present disclosure.

Referring to FIG. 9, the light source unit (110) of the heart rate sensor unit (100) may include an LED (111) and a curved light guide (112).

The light source unit (110) may be centrally formed with the curved light guide (112) having a predetermined radius of curvature to be attached to a wrist area, and may symmetrically (vertically and horizontally) include the first to fourth light receiving units (120, 130, 140, 150) based on a lengthwise direction of the light source unit (110) to allow the LED to come thereon. Each of the first to fourth light receiving units (120, 130, 140, 150) may include the first to fourth photodetectors (121, 131, 141, 151), and a light receiving housing (170) of a bottomless quadrangular pyramid shape.

It is preferable that the heart rate sensor unit (100) be completely attached the skin, but if the heart rate sensor unit (100) is completely attached to the skin, the wearer may feel uncomfortable, such that the connection unit (300) may be in a band type connection unit of elastic material such as silicone and a rubber. Although the band type connection unit may be formed with a space between the band and the attached area with the skin, the heart rate sensor unit (100) according to the present disclosure can emit a sufficient quantity of light using a line light source and four light receiving units to allow measuring an accurate heart rate count.

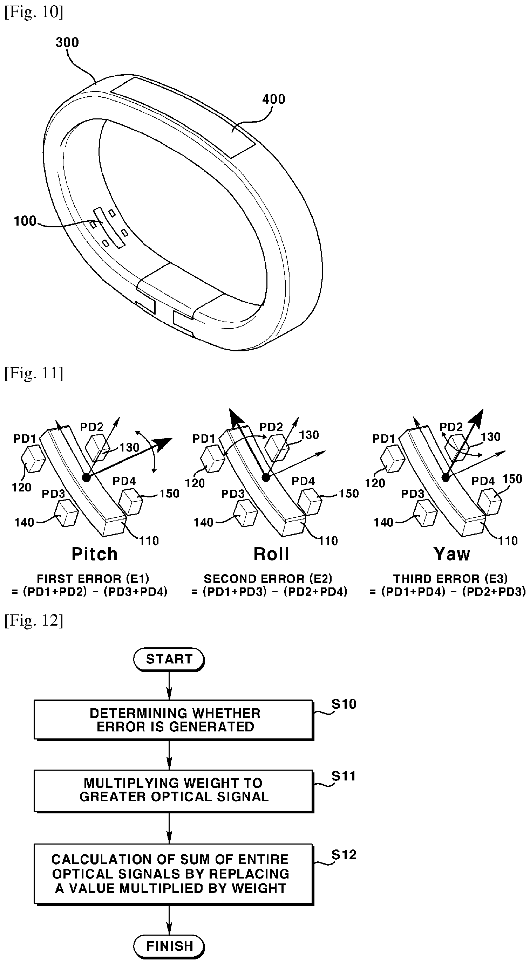

FIG. 10 is a schematic view illustrating a structure of an apparatus for measuring bio-information according to an exemplary embodiment of the present disclosure.

Referring to FIG. 10, the apparatus for measuring bio-information according to an exemplary embodiment of the present disclosure may include a heart rate sensor unit (100), a connection unit (300) and a display unit (400). As illustrated in FIG.1, an acceleration sensor unit (200) may be additionally formed in the apparatus, and other bio-information measuring modules may be further added thereto.

Furthermore, the connection unit (300) may be designed to replace various modules used in the apparatus. Although the connection unit (300) in FIG. 10 is illustrated in the form of a band type connection unit, the present disclosure is not limited thereto, and may be formed in the shape of a wrist watch type connection unit wearable to be attached to a wrist like an wrist watch, and any type wearable to the wrist is also acceptable. The display unit (400) may display various bio-information in addition to a heart rate signal measured by the heart rate sensor unit (100). For example, when a temperature sensor module (not shown) is worn by a wearer, a body temperature may be measured and displayed on the display unit (400).

FIG. 11 is a schematic view illustrating types of alignment errors to be considered when a method for error compensation according to an exemplary embodiment of the present disclosure is performed.

In ideal case, that is, when the heart rate sensor unit is completely attached to the skin, a sum of quantities of lights incident on from each light receiving unit would have a predetermined value. To be more specific, when the heart rate sensor unit is completely attached to the skin, all the light receiving units and the light source unit come to touch the skin to allow a light, which is a subject of detection, to be incident on four light receiving units. Thus, a sum, in which all the quantities of lights incident on the light receiving units are added, may have a predetermined value.

However, when the heart rate sensor unit is not completely attached to the skin to allow some of the light receiving units to be attached to the skin and to allow some of the light receiving units not to be attached to the skin, there may be generated an alignment error. Referring to FIG. 11, quantity of light incident on the first to fourth light receiving units (120, 130, 140, 150) may be respectively defined as a first light receiving signal (PD1), a second light receiving signal (PD2), a third light receiving signal (PD3), and a fourth light receiving signal (PD4).

According to the method for error compensation according to an exemplary embodiment of the present disclosure, when check is made on a sum (PD1+PD2+PD3+PD4) of quantities of lights incident on each light receiving units, a difference (PD1+PD2-(PD3+PD4) between optical signals received by the light receiving units arranged on the upper surface and optical signals received by the light receiving units arranged on the bottom surface, a difference (PD1+PD3-(PD2+PD4)

## Claims

1. An apparatus for measuring bio-information, the apparatus comprising:
a heart rate sensor unit (100) configured to measure heart rates by receiving a light that has entered and come out from skin;
an acceleration sensor unit (200) configured to output a step count by measuring the step count of a wearer;
a display unit (400) configured to display the measured heart rates and step count; and
a wrist-wearable connection unit (300) configured to electrically connect the heart rate sensor, the acceleration sensor unit and the display unit,
**characterized in that** the heart rate sensor unit (100) includes a line light source unit (110), a light receiving unit (120,130,140,150) configured to receive the light that has entered and come out from the skin from the light emitted from the light source unit, and a controller (160) configured to detect the heart rates from a quantity of light received by the light receiving unit,
wherein the light receiving unit (120,130,140,150) includes a first light receiving unit (120) arranged at an upper left surface based on a lengthwise direction of the light source unit, a second light receiving unit (130) arranged at an upper right surface based on a lengthwise direction of the light source unit, a third light receiving unit (140) arranged at a bottom left surface based on a lengthwise direction of the light source unit, and a fourth light receiving unit (150) arranged at a bottom right surface based on a lengthwise direction of the light source unit,
further **characterized in that** the heart rate sensor unit (100) is configured to detect an error by comparing light signals received by the first to fourth light receiving units (120,130,140,150), multiply a predetermined weight to a greater light signal as a result of comparison of the light signals when the error is generated, and add a size of a light signal multiplied by the weight to a size of a light signal not multiplied by the weight.

2. The apparatus of claim 1, wherein the light source unit (110) includes,
an LED (Light Emitting Diode;111) configured to emit a light of point light source shape,
a curved light guide (112) of a particular curvature radius configured to advance the emitted light to a particular direction,
a plurality of V-shaped patterns (113) configured to emit a light to a particular direction by refracting the emitted light, and
a reflective plate (114) configured to reflect a light emitted to an outside from the light guide into an interior of the light guide.

3. The apparatus of claim 2, wherein the LED (111) uses a yellowish green color light source.

4. The apparatus of claim 1, wherein the light receiving unit includes,
a photodetector (121, 131, 141, 151) configured to receive a light that has entered and come out of skin from a light emitted from the light source unit, and
a light receiving housing (170) configured to wrap the photodetector and to gradually broaden at an entrance toward a skin contact surface.

5. The apparatus of claim 2, wherein the plurality of V-shaped patterns (113) tapers off at a spacing of adjacent patterns as being distanced from the LED.

6. The apparatus of claim 1, wherein the connection unit (300) is a wrist band type connector unit of elastic material.

7. The apparatus of claim 1, wherein the connection unit (300) is a wrist watch type, wrist-attachable connector unit.

8. A method for error compensation in a heart rate sensor (100) including a first light receiving unit (120) arranged at an upper left surface of a line light source, a second light receiving unit (130) arranged at an upper right surface of the line light source,
a third light receiving unit (140) arranged at a bottom left surface of the line light source, and a fourth light receiving unit (150) arranged at a bottom right surface of the line light source, the method comprising:
detecting an error by comparing light signals received by the first to fourth light receiving units (120,130,140,150);
multiplying a predetermined weight to a greater light signal as a result of comparison of the light signals when the error is generated; and
adding a size of a light signal multiplied by the weight to a size of a light signal not multiplied by the weight.

9. The method of claim 8, wherein the error includes a first error, which is a difference between a first signal, which is a sum of light signals received by the first light receiving unit (120) and the second light receiving unit (130) and a second signal, which is a sum of light signals received by the third light receiving unit (140) and the fourth light receiving unit (150),
a second error, which is a difference between a third signal, which is a sum of light signals received by the first light receiving unit (120) and the third light receiving unit (140) and a fourth signal, which is a sum of light signals received by the second light receiving unit (130) and the fourth light receiving unit (150), and
a third error, which is a difference between a fifth signal, which is a sum of light signals received by the first light receiving unit (120) and the fourth light receiving unit (150) and a sixth signal, which is a sum of light signals received by the second light receiving unit (130) and the third light receiving unit (140).

10. The method of claim 9, wherein the step of multiplying a predetermined weight to a greater light signal as a result of comparison of the light signals when the error is generated includes,
multiplying a predetermined weight to a greater signal between the first and second signals when the first error is generated,
multiplying a predetermined weight to a greater signal between the third and fourth signals when the second error is generated, and
multiplying a predetermined weight to a greater signal between the fifth and sixth signals when the first error is generated.

11. The method of claim 10, wherein the method further comprises
determining a greater value as a heart rate reference signal between a size of an optical signal multiplied by the weight and a size of an optical signal not multiplied by the weight.

12. A method for error compensation in a heart rate sensor including a first light receiving unit (120) arranged at an upper left surface of a line light source, a second light receiving unit (130) arranged at an upper right surface of the line light source,
a third light receiving unit (140) arranged at a bottom left surface of the line light source, and a fourth light receiving unit (150) arranged at a bottom right surface of the line light source, the method comprising:
detecting an error by comparing light signals received by the first to fourth light receiving units (120,130,140,150);
multiplying a predetermined weight to a light signal having the greatest value as a result of comparison of the light signals when the error is generated; and
adding a size of a light signal multiplied by the weight to a size of a light signal not multiplied by the weight.

13. The method of claim 12, wherein the step of detecting an error by comparing light signals received by the first to fourth light receiving units includes
determining a light receiving unit having received a largest size of optical signal by comparing
a first error, which is a difference between a first signal, which is a sum of light signals received by the first light receiving unit (120) and the second light receiving unit (130) and a second signal, which is a sum of light signals received by the third light receiving unit (140) and the fourth light receiving unit (150),
a second error, which is a difference between a third signal, which is a sum of light signals received by the first light receiving unit (120) and the third light receiving unit (140) and a fourth signal, which is a sum of light signals received by the second light receiving unit (130) and the fourth light receiving unit (150), and
a third error, which is a difference between a fifth signal, which is a sum of light signals received by the first light receiving unit (120) and the fourth light receiving unit (150) and a sixth signal, which is a sum of light signals received by the second light receiving unit (130) and third light receiving unit (140).

## Patentansprüche

1. Vorrichtung zum Messen von Vitalinformationen, wobei die Vorrichtung Folgendes umfasst:
eine Herzfrequenz-Sensoreinheit (100), die konfiguriert ist, Herzfrequenzen durch Empfangen von Licht, das in die Haut eingedrungen und aus dieser ausgetreten ist, zu messen;
eine Beschleunigungssensoreinheit (200), die konfiguriert ist, durch Messen der Schrittzahl eines Trägers eine Schrittzahl auszugeben;
eine Anzeigeeinheit (400), die konfiguriert ist, die gemessenen Herzfrequenzen und die Schrittzahl anzuzeigen; und
eine am Handgelenk tragbare Verbindungseinheit (300), die konfiguriert ist, den Herzfrequenzsensor, die Beschleunigungssensoreinheit und die Anzeigeeinheit elektrisch zu verbinden,
**dadurch gekennzeichnet, dass** die Herzfrequenz-Sensoreinheit (100) eine Linienlichtquelleneinheit (110), eine Lichtempfangseinheit (120, 130, 140, 150), die konfiguriert ist, Licht, das von der Lichtquelleneinheit emittiert wird, in die Haut eingedrungen ist und aus dieser ausgetreten ist, zu empfangen, und eine Steuerung (160), die konfiguriert ist, die Herzfrequenzen aus der Lichtmenge, die von der Lichtempfangseinheit empfangen wird, zu detektieren, umfasst;
wobei die Lichtempfangseinheit (120, 130, 140, 150) eine erste Lichtempfangseinheit (120), die in Bezug auf eine Längsrichtung der Lichtquelleneinheit an einer oberen linken Oberfläche angeordnet ist, eine zweite Lichtempfangseinheit (130), die in Bezug auf eine Längsrichtung der Lichtquelleneinheit an einer oberen rechten Oberfläche angeordnet ist, eine dritte Lichtempfangseinheit (140), die in Bezug auf eine Längsrichtung der Lichtquelleneinheit an einer unteren linken Oberfläche angeordnet ist, und eine vierte Lichtempfangseinheit (150), die n Bezug auf eine Längsrichtung der Lichtquelleneinheit an einer unteren rechten Oberfläche angeordnet ist, umfasst,
ferner **dadurch gekennzeichnet, dass** die Herzfrequenz-Sensoreinheit (100) konfiguriert ist, einen Fehler durch Vergleichen von Lichtsignalen, die von der ersten bis vierten Lichtempfangseinheit (120, 130, 140, 150) empfangen werden, zu detektieren, als ein Ergebnis des Vergleichs der Lichtsignale ein größeres Lichtsignal mit einer festgelegten Gewichtung zu multiplizieren, wenn der Fehler erzeugt wird, und eine Größe eines Lichtsignals, das mit der Gewichtung multipliziert wird, zu einer Größe eines Lichtsignals, das nicht mit der Gewichtung multipliziert wird, zu addieren.

2. Vorrichtung nach Anspruch 1, wobei die Lichtquelleneinheit (110) Folgendes umfasst:
eine LED (Leuchtdiode; 111), die konfiguriert ist, Licht mit einer Punktlichtquellenform zu emittieren,
einen gebogenen Lichtleiter (112) mit einem bestimmten Krümmungsradius, der konfiguriert ist, das emittierte Licht in einer bestimmten Richtung auszubreiten,
mehrere V-förmige Strukturen (113), die konfiguriert sind, Licht in einer bestimmten Richtung zu emittieren, indem das emittierte Licht gebrochen wird, und
eine reflektierende Platte (114), die konfiguriert ist, Licht, das von dem Lichtleiter zu einer Außenseite emittiert wird, in das Innere des Lichtleiters zu reflektieren.

3. Vorrichtung nach Anspruch 2, wobei die LED (111) eine Lichtquelle mit gelblichgrüner Farbe verwendet.

4. Vorrichtung nach Anspruch 1, wobei die Lichtempfangseinheit Folgendes umfasst:
einen Fotodetektor (121, 131, 141, 151), der konfiguriert ist, Licht, das von dem Licht, das von der Lichtquelleneinheit emittiert wird, in die Haut eingedrungen und aus dieser ausgetreten ist, zu empfangen, und
ein Lichtempfangsgehäuse (170), das konfiguriert ist, den Fotodetektor zu umgeben und an einer Öffnung in Richtung einer Hautkontaktfläche schrittweise breiter wird.

5. Vorrichtung nach Anspruch 2, wobei die mehreren V-förmigen Strukturen (113) bei einem Abstand von aneinander angrenzenden Strukturen mit zunehmendem Abstand von der LED schrittweise auseinanderlaufen.

6. Vorrichtung nach Anspruch 1, wobei die Verbindungseinheit (300) eine Verbindungseinheit vom Armbandtyp aus elastischem Material ist.

7. Vorrichtung nach Anspruch 1, wobei die Verbindungseinheit (300) eine Verbindungseinheit vom Armbanduhrtyp ist, die am Handgelenk befestigt werden kann.

8. Verfahren zur Fehlerkompensation in einem Herzfrequenzsensor (100), der eine erste Lichtempfangseinheit (120), die an einer oberen linken Oberfläche einer Linienlichtquelle angeordnet ist, eine zweite Lichtempfangseinheit (130), die an einer oberen rechten Oberfläche der Linienlichtquelle angeordnet ist, eine dritte Lichtempfangseinheit (140), die an einer unteren linken Oberfläche der Linienlichtquelle angeordnet ist, und eine vierte Lichtempfangseinheit (150), die an einer unteren rechten Oberfläche der Linienlichtquelle angeordnet ist, umfasst, wobei das Verfahren die folgenden Schritte umfasst:
Detektieren eines Fehlers durch Vergleichen von Lichtsignalen, die von der ersten bis vierten Lichtempfangseinheit (120, 130, 140, 150) empfangen werden;
Multiplizieren als Ergebnis eines Vergleichs der Lichtsignale eines größeren Lichtsignals mit einer festgelegten Gewichtung, wenn der Fehler erzeugt wird; und
Addieren einer Größe eines Lichtsignals, das mit der Gewichtung multipliziert wird, zu einer Größe eines Lichtsignals, das nicht mit der Gewichtung multipliziert wird.

9. Verfahren nach Anspruch 8, wobei der Fehler die folgenden Fehler umfasst:
einen ersten Fehler, der eine Differenz zwischen einem ersten Signal, das eine Summe von Lichtsignalen ist, die von der ersten Lichtempfangseinheit (120) und der zweiten Lichtempfangseinheit (130) empfangen werden, und einem zweiten Signal ist, das eine Summe der Lichtsignale ist, die von der dritten Lichtempfangseinheit (140) und der vierten Lichtempfangseinheit (150) empfangen werden,
einen zweiten Fehler, der eine Differenz zwischen einem dritten Signal, das eine Summe von Lichtsignalen ist, die von der ersten Lichtempfangseinheit (120) und der dritten Lichtempfangseinheit (140) empfangen werden, und einem vierten Signal ist, das eine Summe von Lichtsignalen ist, die von der zweiten Lichtempfangseinheit (130) und der vierten Lichtempfangseinheit (150) empfangen werden, und
einen dritten Fehler, der eine Differenz zwischen einem fünften Signal, das eine Summe von Lichtsignalen ist, die von der ersten Lichtempfangseinheit (120) und der vierten Lichtempfangseinheit (150) empfangen werden, und einem sechsten Signal ist, das eine Summe von Lichtsignalen ist, die von der zweiten Lichtempfangseinheit (130) und der dritten Lichtempfangseinheit (140) empfangen werden.

10. Verfahren nach Anspruch 9, wobei der Schritt des Multiplizierens eines größeren Lichtsignals mit einer festgelegten Gewichtung als ein Ergebnis eines Vergleichs der Lichtsignale, wenn der Fehler erzeugt wird, die folgenden Schritte umfasst:
Multiplizieren des größeren des ersten und des zweiten Signals mit einer festgelegten Gewichtung, wenn der erste Fehler erzeugt wird,
Multiplizieren des größeren des dritten und des vierten Signals mit einer festgelegten Gewichtung, wenn der zweite Fehler erzeugt wird, und
Multiplizieren des größeren des fünften und des sechsten Signals mit einer festgelegten Gewichtung, wenn der erste Fehler erzeugt wird.

11. Verfahren nach Anspruch 10, wobei das Verfahren ferner den folgenden Schritt umfasst:
Ermitteln des größeren Werts der Größe eines optischen Signals, das mit der Gewichtung multipliziert wurde, und der Größe eines optischen Signals, das nicht mit der Gewichtung multipliziert wurde, als ein Herzfrequenz-Referenzsignal.

12. Verfahren zur Fehlerkompensation in einem Herzfrequenzsensor, der eine erste Lichtempfangseinheit (120), die an einer oberen linken Oberfläche einer Linienlichtquelle angeordnet ist, eine zweite Lichtempfangseinheit (130), die an einer oberen rechten Oberfläche der Linienlichtquelle angeordnet ist, eine dritte Lichtempfangseinheit (140), die an einer unteren linken Oberfläche der Linienlichtquelle angeordnet ist, und eine vierte Lichtempfangseinheit (150), die an einer unteren rechten Oberfläche der Linienlichtquelle angeordnet ist, umfasst, wobei das Verfahren die folgenden Schritte umfasst:
Detektieren eines Fehlers durch Vergleichen von Lichtsignalen, die von der ersten bis vierten Lichtempfangseinheit (120, 130, 140, 150) empfangen werden;
Multiplizieren als ein Ergebnis eines Vergleichs der Lichtsignale eines Lichtsignals, das den größten Wert hat, mit einer festgelegten Gewichtung, wenn der Fehler erzeugt wird; und
Addieren einer Größe eines Lichtsignals, das mit der Gewichtung multipliziert wird, zu einer Größe eines Lichtsignals, das nicht mit der Gewichtung multipliziert wird.

13. Verfahren nach Anspruch 12, wobei der Schritt des Detektierens eines Fehlers durch Vergleichen von Lichtsignalen, die von der ersten bis vierten Lichtempfangseinheit empfangen werden, die folgenden Schritte umfasst:
Ermitteln einer Lichtempfangseinheit, die ein größtes optisches Signal empfangen hat, durch Vergleichen der folgenden Fehler:
einen ersten Fehler, der eine Differenz zwischen einem ersten Signal, das eine Summe von Lichtsignalen ist, die von der ersten Lichtempfangseinheit (120) und der zweiten Lichtempfangseinheit (130) empfangen werden, und einem zweiten Signal, das eine Summe von Lichtsignalen ist, die von der dritten Lichtempfangseinheit (140) und der vierten Lichtempfangseinheit (150) empfangen werden, ist,
einen zweiten Fehler, der eine Differenz zwischen einem dritten Signal, das eine Summe von Lichtsignalen ist, die von der ersten Lichtempfangseinheit (120) und der dritten Lichtempfangseinheit (140) empfangen werden, und einem vierten Signal, das eine Summe von Lichtsignalen ist, die von der zweiten Lichtempfangseinheit (130) und der vierten Lichtempfangseinheit (150) empfangen werden, ist, und
einen dritten Fehler, der eine Differenz zwischen einem fünften Signal, das eine Summe von Lichtsignalen ist, die von der ersten Lichtempfangseinheit (120) und der vierten Lichtempfangseinheit (150) empfangen werden, und einem sechsten Signal, das eine Summe von Lichtsignalen ist, die von der zweiten Lichtempfangseinheit (130) und der dritten Lichtempfangseinheit (140) empfangen werden, ist.

## Revendications

1. Appareil destiné à mesurer des bio-informations, l'appareil comportant :
une unité de capteur de fréquences cardiaques (100) configurée pour mesurer des fréquences cardiaques en recevant une lumière qui est entrée dans la peau et en est ressortie ;
une unité de capteur d'accélération (200) configurée pour délivrer en sortie un nombre de pas en mesurant le nombre de pas d'un porteur ;
une unité d'affichage (400) configurée pour afficher les fréquences cardiaques et le nombre de pas mesurés ; et
une unité de connexion pouvant être portée au poignet (300) configurée pour connecter électriquement l'unité de capteur de fréquences cardiaques, l'unité de capteur d'accélération et l'unité d'affichage,
**caractérisé en ce que** l'unité de capteur de fréquences cardiaques (100) inclut une unité de source de lumière linéaire (110), une unité de réception de lumière (120,130,140,150) configurée pour recevoir la lumière qui est entrée dans la peau et en est ressortie à partir de la lumière émise à partir de l'unité de source de lumière, et une commande (160) configurée pour détecter les fréquences cardiaques à partir d'une quantité de lumière reçue par l'unité de réception de lumière,
dans lequel l'unité de réception de lumière (120,130,140,150) inclut une première unité de réception de lumière (120) agencée sur une surface gauche supérieure basée sur une direction longitudinale de l'unité de source de lumière, une deuxième unité de réception de lumière (130) agencée sur une surface droite supérieure basée sur une direction longitudinale de l'unité de source de lumière, une troisième unité de réception de lumière (140) agencée sur une surface gauche inférieure basée sur une direction longitudinale de l'unité de source de lumière, et une quatrième unité de réception de lumière (150) agencée sur une surface droite inférieure basée sur une direction longitudinale de l'unité de source de lumière,
**caractérisé en outre en ce que** l'unité de capteur de fréquences cardiaques (100) est configurée pour détecter une erreur en comparant des signaux lumineux reçus par les première à quatrième unités de réception de lumière (120,130,140,150), multiplier un poids prédéterminé à un signal lumineux plus élevé en résultat d'une comparaison des signaux lumineux lorsque l'erreur est générée, et ajouter une taille d'un signal lumineux multiplié par le poids à une taille d'un signal lumineux non multiplié par le poids.

2. Appareil selon la revendication 1, dans lequel l'unité de source de lumière (110) inclut :
une LED (diode électroluminescente ; 111) configurée pour émettre une lumière en forme de source de lumière ponctuelle,
un conduit de lumière incurvé (112) ayant un rayon de courbure particulier configuré pour faire avancer la lumière émise dans une direction particulière,
une pluralité de motifs en forme de V (113) configurés pour émettre une lumière dans une direction particulière en réfractant la lumière émise, et
une plaque réfléchissante (114) configurée pour réfléchir une lumière émise vers un extérieur à partir du conduit de lumière dans un intérieur du conduit de lumière.

3. Appareil selon la revendication 2, dans lequel la LED (111) utilise une source de lumière de couleur vert jaunâtre.

4. Appareil selon la revendication 1, dans lequel l'unité de réception de lumière inclut :
un photodétecteur (121, 131, 141, 151) configuré pour recevoir une lumière qui est entrée dans la peau et en est ressortie à partir d'une lumière émise à partir de l'unité de source de lumière, et
un boîtier de réception de lumière (170) configuré pour envelopper le photodétecteur et pour s'élargir graduellement au niveau d'une entrée vers une surface en contact avec la peau.

5. Appareil selon la revendication 2, dans lequel les motifs adjacents de la pluralité de motifs en forme de V (113) se rapprochent en termes d'espacement à mesure qu'ils s'éloignent de la LED.

6. Appareil selon la revendication 1, dans lequel l'unité de connexion (300) est une unité de connexion de type bracelet de poignet en matériau élastique.

7. Appareil selon la revendication 1, dans lequel l'unité de connexion (300) est une unité de connexion de type montre-bracelet, attachable au poignet.

8. Procédé de compensation d'erreur dans un capteur de fréquences cardiaques (100) incluant une première unité de réception de lumière (120) agencée sur une surface gauche supérieure d'une source de lumière linéaire, une deuxième unité de réception de lumière (130) agencée sur une surface droite supérieure de la source de lumière linéaire,
une troisième unité de réception de lumière (140) agencée sur une surface gauche inférieure de la source de lumière linéaire, et une quatrième unité de réception de lumière (150) agencée sur une surface droite inférieure de la source de lumière linéaire, le procédé comportant les étapes consistant à :
détecter une erreur en comparant des signaux lumineux reçus par les première à quatrième unités de réception de lumière (120, 130, 140, 150) ;
multiplier un poids prédéterminé à un signal lumineux plus élevé en résultat d'une comparaison des signaux lumineux lorsque l'erreur est générée ; et
ajouter une taille d'un signal lumineux multiplié par le poids à une taille d'un signal lumineux non multiplié par le poids.

9. Procédé selon la revendication 8, dans lequel l'erreur inclut une première erreur, qui est une différence entre un premier signal, qui est une somme de signaux lumineux reçus par la première unité de réception de lumière (120) et la deuxième unité de réception de lumière (130), et un second signal, qui est une somme de signaux lumineux reçus par la troisième unité de réception de lumière (140) et la quatrième unité de réception de lumière (150),
une deuxième erreur, qui est une différence entre un troisième signal, qui est une somme de signaux lumineux reçus par la première unité de réception de lumière (120) et la troisième unité de réception de lumière (140), et un quatrième signal, qui est une somme de signaux lumineux reçus par la deuxième unité de réception de lumière (130) et la quatrième unité de réception de lumière (150), et
une troisième erreur, qui est une différence entre un cinquième signal, qui est une somme de signaux lumineux reçus par la première unité de réception de lumière (120) et la quatrième unité de réception de lumière (150), et un sixième signal, qui est une somme de signaux lumineux reçus par la deuxième unité de réception de lumière (130) et la troisième unité de réception de lumière (140).

10. Procédé selon la revendication 9, dans lequel l'étape consistant à multiplier un poids prédéterminé à un signal lumineux plus élevé en résultat d'une comparaison des signaux lumineux lorsque l'erreur est générée inclut les étapes consistant à :
multiplier un poids prédéterminé à un signal plus élevé entre les premier et deuxième signaux lorsque la première erreur est générée,
multiplier un poids prédéterminé à un signal plus élevé entre les troisième et quatrième signaux lorsque la deuxième erreur est générée, et
multiplier un poids prédéterminé à un signal plus élevé entre les cinquième et sixième signaux lorsque la première erreur est générée.

11. Procédé selon la revendication 10, dans lequel le procédé comporte en outre l'étape consistant à :
déterminer une valeur plus élevée comme étant un signal de référence de fréquence cardiaque entre une taille d'un signal optique multiplié par le poids et une taille d'un signal optique non multiplié par le poids.

12. Procédé de compensation d'erreur dans un capteur de fréquences cardiaques incluant une première unité de réception de lumière (120) agencée sur une surface gauche supérieure d'une source de lumière linéaire, une deuxième unité de réception de lumière (130) agencée sur une surface droite supérieure de la source de lumière linéaire,
une troisième unité de réception de lumière (140) agencée sur une surface gauche inférieure de la source de lumière linéaire, et une quatrième unité de réception de lumière (150) agencée sur une surface droite inférieure de la source de lumière linéaire, le procédé comportant les étapes consistant à :
détecter une erreur en comparant des signaux lumineux reçus par les première à quatrième unités de réception de lumière (120, 130, 140, 150) ;
multiplier un poids prédéterminé à un signal lumineux ayant la plus grande valeur en résultat d'une comparaison des signaux lumineux lorsque l'erreur est générée ; et
ajouter une taille d'un signal lumineux multiplié par le poids à une taille d'un signal lumineux non multiplié par le poids.

13. Procédé selon la revendication 12, dans lequel l'étape de détection d'une erreur en comparant des signaux lumineux reçus par les première à quatrième unités de réception de lumière inclut les étapes consistant à :
déterminer une unité de réception de lumière ayant reçu la plus grande taille de signal optique en comparant
une première erreur, qui est une différence entre un premier signal, qui est une somme de signaux lumineux reçus par la première unité de réception de lumière (120) et la deuxième unité de réception de lumière (130), et un second signal, qui est une somme de signaux lumineux reçus par la troisième unité de réception de lumière (140) et la quatrième unité de réception de lumière (150),
une deuxième erreur, qui est une différence entre un troisième signal, qui est une somme de signaux lumineux reçus par la première unité de réception de lumière (120) et la troisième unité de réception de lumière (140), et un quatrième signal, qui est une somme de signaux lumineux reçus par la deuxième unité de réception de lumière (130) et la quatrième unité de réception de lumière (150), et
une troisième erreur, qui est une différence entre un cinquième signal, qui est une somme de signaux lumineux reçus par la première unité de réception de lumière (120) et la quatrième unité de réception de lumière (150), et un sixième signal, qui est une somme de signaux lumineux reçus par la deuxième unité de réception de lumière (130) et la troisième unité de réception de lumière (140).
